# EUROPEAN PATENT SPECIFICATION

(11) **EP 1 490 510 B1**
(45) Date of publication and mention of the grant of the patent: **14.05.2008**
(21) Application number: 02717009.1
(22) Date of filing: 26.03.2002
(51) Int. Cl.: C12Q 1/68

(54) **NOVEL PRIMERS FOR IDENTIFYING AFLATOXINOGENIC ASPERGILLI AND AN IMPROVED USE THEREOF**
NEUE PRIMER ZUR IDENTIFIZIERUNG VON AFLATOXINOGENEN ASPERGILLI UND DEREN VERBESSERTE VERWENDUNGEN
NOUVELLES AMORCES UTILES POUR IDENTIFIER LES ASPERGILLUS AFLATOXINOGENES ET UTILISATION AMELIOREE DE CES DERNIERES

(43) Date of publication of application: 29.12.2004
(73) Proprietor: COUNCIL OF SCIENTIFIC AND INDUSTRIAL RESEARCH, New Delhi 110 001 (IN)
(72) Inventor: MANONMANI, Haravey Krishnan, Mysore, Karnataka 570 013 (IN); CHANDRASHEKAR, Arun, Mysore, Karnataka 570 013 (IN); RAO, Eddiya Rati, Mysore, Karnataka 570 013 (IN)
(74) Representative: Bloch, Gérard
(86) International application number: PCT/IB2002/001162
(87) International publication number: WO 2003/080866

(56) References cited:
- EP-A- 0 860 503
- WO-A-00/73499
- US-A- 5 707 802
- SHAPIRA RONI ET AL: "Detection of aflatoxigenic molds in grains by PCR." APPLIED AND ENVIRONMENTAL MICROBIOLOGY, vol. 62, no. 9, 1996, pages 3270-3273, XP002224638 ISSN: 0099-2240 cited in the application
- VAN DEN BROEK P ET AL: "Aflatoxin genes and the aflatoxigenic potential of Koji moulds." APPLIED MICROBIOLOGY AND BIOTECHNOLOGY. GERMANY OCT 2001, vol. 57, no. 1-2, October 2001 (2001-10), pages 192-199, XP002224639 ISSN: 0175-7598
- FAERBER PAUL ET AL: "Detection of aflatoxinogenic fungi in figs by a PCR reaction." INTERNATIONAL JOURNAL OF FOOD MICROBIOLOGY, vol. 36, no. 2-3, 1997, pages 215-220, XP002224640 ISSN: 0168-1605
- YU J ET AL: "Genes encoding cytochrome P450 and monooxygenase enzymes define one end of the aflatoxin pathway gene cluster in Aspergillus parasiticus." APPLIED MICROBIOLOGY AND BIOTECHNOLOGY, vol. 53, no. 5, May 2000 (2000-05), pages 583-590, XP001128784 ISSN: 0175-7598
- BRETAGNE S ET AL: "DETECTION OF ASPERGILLUS SPECIES DNA IN BRONCHOALVEOLAR LAVAGE SAMPLES BY COMPETITIVE PCR" JOURNAL OF CLINICAL MICROBIOLOGY, WASHINGTON, DC, US, vol. 33, no. 5, May 1995 (1995-05), pages 1164-1168, XP000874668 ISSN: 0095-1137
- MAKIMURA K ET AL: "DETECTION OF A WIDE RANGE OF MEDICALLY IMPORTANT FUNGI BY THE POLYMERASE CHAIN REACTION" JOURNAL OF MEDICAL MICROBIOLOGY, HARLOW, GB, vol. 40, no. 5, 1 May 1994 (1994-05-01), pages 358-364, XP002046486 ISSN: 0022-2615

## Description

### Technical Field

The present invention relates to a set of novel primers of SEQ ID Nos. 1-2, wherein said set of primer is designed from gene *omt*, of aflatoxin biosynthesis pathway of fungi *Aspergillus flavus* and an improved use of identifying aflatoxinogenic *aspergilli* using said set of primers.

### Background Art

Aflatoxins are potent carcinogenic, mutagenic and teratogenic metabolites produced primarily by the fungal species of *Aspergillus flavus* and *Aspergillus parasiticus*. Foods and feeds, especially in warm climates are susceptible to invasion by aflatoxigenic *Aspergillus* sp. And subsequent production of Aflatoxins during preharvesting, processing, transportation or storage. Over the last few years, means for mycotoxin detection have been simplified, by the adoption of immunological methods. The level of mold infestation and identification of the governing species are important parameters which could give an indication of the quality of the material and future potential for the presence of mycotoxins.

Mold counts are a part of quality control assurance for foods. This use is time consuming, labour intensive , costly requires facilities and mycological expertise and do not allow the specification of mycotoxegenic fungi.

With the advances made in the detection methods, polymerase chain reaction (PCR) facilitates *in vitro* amplification of target sequence and offers several advantages over traditional methods of detection.

Reference may be made to the work of Miller and Martin (1988) for the application of PCR techniques for the detection of microorganisms, including plant pathogens. However no attempt has been made to detect aflatoxin-producing fungi.

Reference may be made to the works of Payne and Woloshuk (1989) and Nu, *et al* (1995). Who have identified the genes in Aflatoxins biosynthetic pathway of strains of *A. flavus* and *A. parasiticus*. However, detection of aflatixigenic fungi were not attempted.

Reference may be made to the work of Shapira, et al (1996) , where in the identification of aflatoxin producing molds in grains has been attempted using PCR techniques. Three genes *ver* - 1, *omt -* 1 and *apa -* 2 coding for key enzymes and regulatory factor in biosynthesis of aflatoxin were used as primers. Positive results were obtained in 24h enriched cultures at lowest spore level of 10² spores per gram. However incubation of dried ground corn seeds in enrichment media allowed detection as few as 10² spores per gram after 48h of incubation.

Other known references to methods for detecting fungi belonging to the genes Aspergillus are the following:
EP-A-0 860 503 (SS PHARMACEUTICAL CO.) 26 August 1998 YU J ET AL: 'Genes encoding cytochrome P450 and monooxygenase enzymes define one end of the aflatoxin pathway gene cluster in Aspergillus parasiticus.' APPLIED MICROBIOLOGY AND BIOTECHNOLOGY, vol. 53, no. 5, May 2000 (2000-05), pages 583-590.
FAERBER PAUL ET AL: 'Detection of aflatoxinogenic fungi in figs by a PCR reaction. INTERNATIONAL JOURNAL OF FOOD MICROBIOLOGY, vol. 36, no. 2-3, 1997, pages 215-220, XP002224640 ISSN: 0168-1605
SHAPIRA RONI ET AL: 'Detection of aflatoxigenic molds in grains by PCR.' APPLIED AND ENVIRONMENTAL MICROBIOLOGY, vol. 62, no. 9, 1996, pages 3270-3273, XP002224638 ISSN: 0099-2240, cited in the application.

The traditional methods are non-sensitive, time consuming and lack consistency. The present invention enables detection of aflatoxigenic fungi by PCR using specific aflatoxin biosynthetic pathway genes. This use has application in food system.

### Objects of the present invention

The main object of the present invention is to develop primers from genes of aflatoxin biosynthesis pathway.

Another main object of the present invention is to develop primers for gene *omt*, of aflatoxin biosynthesis pathway.

Yet another object of the present invention is to develop an improved use for identifying aflatoxinogenic aspergilli.

Still another object of the present invention is to develop an improved use for identifying aflatoxinogenic aspergilli using said primers.

Still another object of the present invention is to develop a use of identifying aflatoxinogenic aspergilli directly in food articles.

Still another object of the present invention is to develop a highly sensitive use to identifying aflatoxinogenic aspergilli.

Further object of the present invention is to develop an identification use using multiple number of primers for more accuracy.

### Summary of the present invention

The present invention relates to a set of novel primers of SEQ ID Nos. 1-2, wherein said set of primer is designed from gene *omt*, of aflatoxin biosynthesis pathway of fungi *Aspergillus flavus* and an improved use of identifying aflatoxinogenic *aspergilli* using said set of primers.

### Detailed description of the present invention

Accordingly, the present invention relates to a set of novel primers of SEQ ID Nos. 1-2, wherein said three sets of primer are designed from gene *omt,* of aflatoxin biosynthesis pathway of fungi *Aspergillus flavus* and an improved use of identifying aflatoxinogenic *aspergilli* using said set of primers.

In one embodiment of the present invention, a set of oligonucleotide primers of SEQ ID Nos. 1 through 2. (Please refer sequences shown below).
*omt* 1 (F) 5'AGCGTCCGAATCCCTTTAAT 3' (SEQ ID NO. 1)
(R) 5' AGGGTGTTCGCCAATCATAG 3' (SEQ ID NO. 2)

In further embodiment of the present invention, three sets of primer of SEQ ID Nos. 1-6 used together shows more accurate identification as compared to one set of primer alone.

| | |
|---|---|
| *ord* | (F) 5' ACTGCCCCTCAGCTAACCTC 3' (SEQ ID NO.3) |
| | (R) 5' GCATCAGCATTCTTCCAAGG 3 (SEQ ID NO. 4) |

| | |
|---|---|
| *aflR* | (F) 5' AACCGCATCCACA ATCTCAT 3 (SEQ ID NO.5) |
| | (R) 5' AGTGCAGTTCGCTCAGAACA3 (SEQ ID NO. 6) |

In another embodiment of the present invention, wherein said primers are designed from genes of aflatoxin biosynthesis pathway of fungi *Aspergillus flavus*.

In yet another embodiment of the present invention, wherein said primers are designed for specific genes *omt.*

In still another embodiment of the present invention, wherein primers 1 and 2 correspond to gene *omt* encoding o-methyl transferase.

primers 3 and 4 correspond to gene *ord* encoding oxidoreductase.

primers 5 and 6 correspond to gene *afl* R encoding aflatoxin regulatory protein.

primer 1, is forward primer.

primer 2, is reverse primers.

In still another embodiment of the present invention, wherein length of primers is 20 base pairs (bp).

Sequences of three genes omt, ordA, and afl R are as follows:
**A.** O methyltransferase (Omt) gene from the published gene sequence with Accession no L25834 where the primers covers the region between 1811 to 2218 with the product size 407 bp of SEQ ID NO. 7.
**B.** Oxidoreductase (ord) gene from the published gene sequence with Accession no AF 169016 where the primer covers the region between 3142 to 3530 with product size 388 bp of SEQ ID NO. 8.
**C.** Aflotoxin regulatory gene (aflR) from the published gene sequence with Accession no AF 264763 where the said primer covers the region between 540 to 1338 with the product size 798 bp of SEQ ID NO. 9.

In further embodiment of the present invention, an improved method of identifying aflatoxigenic *aspergilli* using primers of claim 1 in combination, comprising steps of :
a. Harvesting mixed microflora from food system,
b. Extracting DNA from said harvested flora,
c. Amplifying DNA by PCR using said primers,
d. Analyzing amplified DNA by electrophoresis, and
e. Identifying aflatoxigenic fungi.

### Brief description of the accompanying drawings

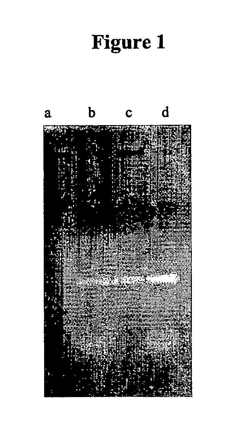

**Figure 1** shows amplicons from the PCR with three sets of primers from DNA of *Aspergillus flavus* ATCC 46283. a, b, c, d,, DNA isolated from samples harvested at 12,24,36 and 48 hours and amplified with primers for the *afl*R gene

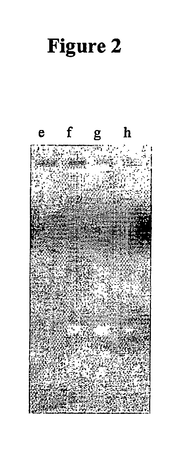

**Figure 2** shows amplicons from the PCR with three sets of primers from DNA of *Aspergillus flavus* ATCC 46283. e, f, g, h DNA isolated from samples harvested at 12,24,36 and 48 hours and amplified with primers for the *omt* gene

In still another embodiment of the present invention, wherein fungi are harvested by centrifugation.

In still another embodiment of the present invention, wherein DNA is extracted using mixture of phenol, chloroform, and amyl alcohol in ratio of about 25:24:1.

In still another embodiment of the present invention, wherein primers are designed using software programme Primer 3.0.

In still another embodiment of the present invention, wherein primers 1 and 2 amplify 406 base pairs (bp) fragment of gene *omt.*

primers 3 and 4 amplify 387 base pairs (bp) fragment of gene *ord*.

primers 5 and 6 amplify 1299 base pairs (bp) fragment of gene *afl* R.

In still another embodiment of the present invention, wherein amplification mixture for amplifying DNA comprises Tris-HCl, Potassium Chloride (KCl), Magnesium Chloride (MgCl₂), gelatin, deoxyribonucleoside triphosphates, primers, *Taq* DNA polymerase, and sterile ultra filtered water.

In still another embodiment of the present invention, wherein electrophorizing amplified DNA using agarose gel.

In still another embodiment of the present invention, wherein said use identifies aflatoxigenic fungi as young as 12 hrs old.

In still another embodiment of the present invention, wherein said use is particularly useful in detecting aflatoxigenic *Aspergillus flavus*, and *Aspergillus parasiticus*.

In still another embodiment of the present invention, wherein said use detects aflatoxigenic fungi directly in food grains.

In still another embodiment of the present invention, wherein said use detects aflatoxigenic fungi from 10⁻² to 10⁻⁶ cell numbers in grains.

In still another embodiment of the present invention, wherein said use extracts DNA without using liquid nitrogen.

In still another embodiment of the present invention, said use with three sets of primer use together shows more accurate identification as compared to any one set of primer alone. Accordingly, the present invention provides an improved method for the detection of aflatoxigenic molds in food systems according to the claims which may optionally further comprise:
a). Designing a set of nucleotide primers for *o*-methyl transferase, oxidoreductase and aflatoxin regulatory genes in *Aspergillus flavus*

| | |
|---|---|
| *omt* 1 | (F) 5' AGCGTCCGAATCCCTTTAAT 3' (SEQ ID NO. 1) |
| | (R) 5' AGGGTGTTCGCCAATCATAG 3' (SEQ ID NO. 2) |

| | |
|---|---|
| *ord* | (F) 5' ACTGCCCCTCAGCTAACCTC 3' (SEQ ID NO. 3) |
| | (R) 5' GCATCAGCATTCTTCCAAGG 3' (SEQ ID NO. 4) |

| | |
|---|---|
| *aflR* | (F) 5' AACCGCATCCACA ATCTCAT 3' (SEQ ID NO. 5) |
| | (R) 5' AGTGCAGTTCGCTCAGAACA3 (SEQ ID NO. 6) |

b). A use for detection of aflatoxigenic fungi using primers specific for *omt, ord* and *afl* R *gene* in a mixed microflora.
c). Extraction of template DNA from moulds in grains or other food commodities.
d). Extraction of template DNA from *Aspergillus flavus, Aspergillus parasiticus* and *Fusarium* sp. may be effected by grinding the fungal mass in Tris - EDTA (50mM: 5mM) buffer containing % sodium dodocyl sulphate ( SDS ), followed by extraction using phenol : iso- amyl alcohol at 25 : 24 : 0.1.
e). The PCR reaction mixture in a total volume of 25 µl may consist of buffer 2.5µl, d NTP mix 0.5 µl, taq - polymerase 0.3 µl, water 18.78 µl, each specific primer - forward 1µl, reverse 1 µl and template DNA 1µl.
f). Detection of aflatoxigenic fungi by amplification of target gene may be effected from an initial denaturation at 90° - 98°C for 2 - 8min, amplification cycle of 28 to 40 each cycle with a denaturation at 90° - 98°C for 40 - 70secs,annealing at 46° - 62°C for 40 - 80secs and an extension at 68° - 76°C for 4 to 12min.
g). The analysis of the PCR product may be achieved in 1.2 - 1.8% agarose gel electrophoresis, visualization of PCR product by staining with 0.5 µg /ml ethidium bromide and observation in a UV - transilluminator.
i). Detection of time course may be effected indicating the rapidity of detection.
j). Detection of aflatoxigenic moulds amongst different moulds may be effected indicating high sensitivity of the reaction.

In an embodiment of the present invention, effective amplification of *o*-methyl transferase, oxidoreductase and aflatoxin regulatory genes may be effected at initial denaturation at 93° - 95°C for 4 - 6min, amplification cycles of 32 - 38, each cycle with a denaturation at 93° - 95°C for 55 - 65 seconds annealing at 48° - 52°C for 55 - 65 seconds and an extension at 70° - 74°C for 55 - 65 seconds and a final extension at 68° - 76°C for 6 - 10min.

In another embodiment of the present invention, the PCR use can detect from 12 to 120h old mycelia.

In yet another embodiment of the present invention, the PCR use may detect toxigenic fungi directly in grains.

The patent relates to PCR use for the detection of aflatoxigenic fungi. Polymerase chain reaction use was used to selectively amplify *o*-methyl transferase, oxidoreductase and aflatoxin regulatory genes in toxigenic fungi. Fungi grown in czapek-dox or potato dextrose broth or from contaminated grains were used for the isolation of template DNA.

The PCR reaction mixture and amplification conditions were optimized for specific amplification. Visualization of PCR products revealed that by the use followed, it is possible to detect toxigenic fungi from 10⁻² to 10⁻⁶ cell numbers in grains and only from aflatoxigenic fungi.

The novelty of this use is the use of the designed primers for the direct detection of aflatoxigenic moulds by PCR. This use can detect aflatoxigenic fungi from contaminated grains. The use is rapid and sensitive making it possible to detect aflatoxigenic fungi from contaminated food systems without culturing them.

The following examples are given by way of illustrations of the present invention and therefore should not be construed to limit the scope of the present invention.

### Example 1

Oligonucleotide primers for o-methyltransferase, oxidoreductase, and aflatoxin regulatory gene c *Aspergillus flavus* were designed based on the gene sequence (ENTREZ) using the softward programme primer 3.0. These primer sets amplify 406, 387and 1299 base pair (bp) respectively fragment of the gene, the sequence of which is given below. Sterilization of media and othe solutions was achieved by autoclaving for 20 min. at 121°C.

| | |
|---|---|
| *omt* 1 | (F) 5' AGCGTCCGAATCCCTTTAAT 3' |
| *omt* 2 | (R) 5' AGGGTGTTCGCCAATCATAG 3' |

| | |
|---|---|
| *ord* 1 | (F) 5' ACTGCCCCTCAGCTAACCTC 3' |
| *ord* 2 | (R) 5' GCATCAGCATTCTTCCAAGG 3' |

| | |
|---|---|
| *aflR* 1 | (F) 5' AACCGCATCCACA ATCTCAT 3' |
| *afl* R 2 | (R) 5 AGTGCAGTTCGCTCAGAACA3 |

Ten ml of Czapek-dox or potato dextrose broth was inoculated with 1 ml spore suspension of *Aspergillus flavus* ATCC 46283, *Aspergillus ochraceus* CFR 221 and *Fusarium tricinctum* NRRL 32998 separately and the flasks were incubated for 120h at ambient temperature (26° - 28°C) under stationary conditions. The mycelia were harvested by centrifugation, washed well with buffer, ground well using lysis buffer after freezing in liquid nitrogen. After 1h of incubation at 65°C, DNA was extracted using phenol: chloroform: amyl alcohol mixture at 25:24:1 concentration. The aqueous phase was redissolved in Tris-EDTA buffer.

Amplification was performed in a total reaction volume of 25µl which contained 1 x PCR buffer (10 mM Tris-HCl, pH 9.0, 50 mM KCl, 1.5 mM MgCl₂, 0.01% gelatin), each of deoxyribonucleoside triphosphate,4nm of each primer and unit of taq DNA polymerase and sterile ultra filtered water. Template DNAs were initially denatured at 94°C for 4 min. Subsequently, a total of 35 amplification cycle was carried out in a programmable thermocycler. Each cycle consisted of denaturation for 30 sec. at 94°C, primer annealing for 45 secs.at 50°C, 58°C and 62°C separately and an extension for 1.15 min. at 72°C. The last cycle was followed by a final extension at 72°C for 10 min.

PCR products were analysed by agarose gel electrophoresis. Aliquots of 10µl PCR products were mixed with 2.0 µl of loading dye and loaded on to 1.2% agarose gel and subjected to electrophoresis for 2h at 100 volts in 1 x TAE buffer. Gel was stained with ethidium bromide (0.5µg /ml), destained with distilled water and examined on a UV transilluminator. A 100 bp ladder was used as a molecular size marker. The amplification profile in the gel was documented in a CCD-camera based gel documentation system.

Specific amplicons of 406, 387, and 1299 bp for *omt, ord* and *afl* R were observed with *Aspergillus flavus* template DNA while, *Aspergillus ochraceus* and *Fusarium tricinctum* template genes did not show amplification.

### Example 2

Oligonucleotide primers for o-methyltransferase, oxidoreductase, and aflatoxin regulatory gene of *Aspergillus flavus* were designed based on the gene sequence (ENTREZ) using the software programme primer 3.0. These primer sets amplify 406, 387, and 1299 base pair (bp) fragment of the gene, the sequence of which is given below. Sterilization of media and other solutions was achieved by autoclaving for 20 min. at 121 °C.

| | |
|---|---|
| *omt* 1 | (F) 5' AGCGTCCGAATCCCTITAAT 3' |
| *omt 2* | (R) 5' AGGGTGTTCGCCAATCATAG 3' |

| | |
|---|---|
| *ord* 1 | (F) 5' ACTGCCCCTCAGCTAACCTC 3' |
| *ord* 2 | (R) 5' GCATCAGCATTCTTCCAAGG 3' |

| | |
|---|---|
| *aflR* 1 | (F) 5' AACCGCATCCACA ATCTCAT 3' |
| *afl* R 2 | (R) 5' AGTGCAGTTCGCTCAGAACA3' |

Ten ml of Czapek-dox or potato dextrose broth was inoculated with 1 ml spore suspension of *Aspergillus flavus* ATCC 46283 and the flasks were incubated for 120h at ambient temperature (26° - 28°C) under stationary conditions. The mycelia were harvested by centrifugation, washed well with buffer, ground well using lysis buffer after freezing in liquid nitrogen. After 1h of incubation at 65°C, DNA was extracted using phenol : chloroform : amyl alcohol mixture at 25:24:1 concentration. The aqueous phase was redissolved in Tris-EDTA buffer
Amplification was performed in a total reaction volume of 25µl which contained I x PCR buffer (10 mM Tris-HCl, pH 9.0, 50 mM KCl, 1.5 mM MgCl₂, 0.01% gelatin), each of deoxyribonucleoside triphosphate,4nm of each primer and unit of taq DNA polymerase and sterile ultra filtered water. Template DNAs were initially denatured at 94°C for 4 min. Subsequently, a total of 35 amplification cycle was carried out in a programmable thermocycler. Each cycle consisted of denaturation for 30 sec. at 94°C, primer annealing for 45 secs.at 50°C, 58°C and 62°C separately and an extension for 1.15 min. at 72°C.

The last cycle was followed by a final extension at 72°C for 10 min.

PCR products were analysed by agarose gel electrophoresis. Aliquots of 10µl PCR products were mixed with 2.0 µl of loading dye and loaded on to 1.2% agarose gel and subjected to electrophoresis for 2h at 100 volts in 1 x TAE buffer. Gel was stained with ethidium bromide (0.5µg /ml), destained with distilled water and examined on a UV transilluminator. A 100 bp ladder was used as a molecular size marker. The amplification profile in the gel was documented in a CCD-camera based gel documentation system.

At 50°C amplification was observed in all 3 primers. At 55°C *omt* did not show any amplicons. However for *ord* and *afl* R primers the amplicons were observed. At 62°C all the primers showed no amplicons production.

### Example 3

Oligonucleotide primers for *o*-methyltransferase, oxidoreductase, and aflatoxin regulatory gene of *Aspergillus flavus* were designed based on the gene sequence (ENTREZ) using the software programme primer 3.0. These primer sets amplify 406, 387, and 1299 base pair (bp) fragment of the gene, the sequence of which is given below. Sterilization of media and other solutions was achieved by autoclaving for 20 min. at121 °C.

| | |
|---|---|
| *omt* 1 | (F) 5' AGCGTCCGAATCCCTTTAAT 3' |
| *omt* 2 | (R) 5' AGGGTGTTCGCCAATCATAG 3' |

| | |
|---|---|
| *ord* 1 | (F) 5' ACTGCCCCTCAGCTAACCTC 3' |
| *ord* 2 | (R) 5' GCATCAGCATTCTTCCAAGG 3' |

| | |
|---|---|
| *aflR* 1 | (F) 5' AACCGCATCCACA ATCTCAT 3' |
| *af* R | 2 (R) 5' AGTGCAGTTCGCTCAGAACA3' |

Ten ml of Czapek-dox or potato dextrose broth was inoculated with 1 ml spore suspension of *Aspergillus flavus* ATCC 46283 and the flasks were incubated for 120h at ambient temperature (26° - 28°C) under stationary conditions. The mycelia were harvested at different periods from 12h through 144h by centrifugation, washed well with buffer, ground well using lysis buffer after freezing in liquid nitrogen. After 1h of incubation at 65°C, DNA was extracted using phenol : chloroform : amyl alcohol mixture at 25:24:1 concentration. The aqueous phase was redissolved in Tris-EDTA buffer.

Amplification was performed in a total reaction volume of 25µl which contained 1 x PCR buffer (10 mM Tris-HCl, pH 9.0, 50 mM KCl, 1.5 mM MgCl₂, 0.01% gelatin), each of deoxyribonucleoside triphosphate,4nM of each primer and unit of taq DNA polymerase and sterile ultra filtered water. Template DNAs were initially denatured at 94°C for 4 min. Subsequently, a total of 35 amplification cycle was carried out in a programmable thermocycler. Each cycle consisted of denaturation for 30 sec. at 94°C, primer annealing for 45 sees at 50°C, and an extension for 1.15 min. at 72°C. The last cycle was followed by a final extension at 72°C for 10 min.

PCR products were analysed by agarose gel electrophoresis. Aliquots of 10µl PCR products were mixed with 2.0 µl of loading dye and loaded on to 1.2% agarose gel and subjected to electrophoresis for 2h at 100 volts in 1 x TAE buffer. Gel was stained with ethidium bromide (0.5µg /ml), destained with distilled water and examined on a UV transilluminator. A 100 bp ladder was used as a molecular size marker. The amplification profile in the gel was documented in a CCD-camera based gel documentation system. Amplification could be observed from DNA extracted from 24h to 120h grown mycelia. Amplifications were observed with *omt, ord* and *afl* R primers.

### Example 4

Oligonucleotide primers for *o*-methyltransferase, oxidoreductase, and aflatoxin regulatory gene of *Aspergillus flavus* were designed based on the gene sequence (ENTREZ) using the software programme primer 3.0. These primer sets amplify 406, 387, and 1299 base pair (bp) fragment of the gene, the sequence of which is given below. Sterilization of media and other solutions was achieved by autoclaving for 20 min. at121 °C.

| | |
|---|---|
| *omt* 1 | (F) 5' AGCGTCCGAATCCCTTTAAT 3' |
| *omt* 2 | (R) 5' AGGGTGTTCGCCAATCATAG 3' |

| | |
|---|---|
| *ord* 1 | (F) 5' ACTGCCCCTCAGCTAACCTC 3' |
| *ord 2* | (R) 5' GCATCAGCATTCTTCCAAGG 3' |

| | |
|---|---|
| *aflR* 1 | (F) 5; AACCGCATCCACA ATCTCAT 3' |
| *aflR* 2 | (R) 5' AGTGCAGTTCGCTCAGAACA3" |

One ml spore suspensions of 27 different fungi, belonging to Fusarium spp (6 no.)., *Aspergillus flavus* (6 nos.), *Aspergillus parasiticus*.(3nos.), Aspergillus spp, (4 nos.), *Aspergillus oryzae ( 3 nos.),Rhizopus* spp (3 nos.) were inoculated to potato - dextrose broth and incubated at ambient temperatures (26° - 28°C) under stationary conditions for 96h. DNA was extracted from ground mycelia without liquid nitrogen treatment.

Amplification was performed in a total reaction volume of 25µl which contained 1 x PCR buffer (10 mM Tris-HCl, pH 9.0, 50 mM KCl, 1.5 mM MgCl₂, 0.01% gelatin), each of deoxyribonucleoside triphosphate,4nm of each primer and unit of taq DNA polymerase and sterile ultra filtered water. Template DNAs were initially denatured at 94°C for 4 min. Subsequently, a total of 35 amplification cycles were carried out in a programmable thermocycler. Each cycle consisted of denaturation for 30 sec. At 94°C, primer annealing for 45 secs.at 50°C and an extension for 1.15 min. at 72°C. The last cycle was followed by a final extension at 72°C for 10 min.

PCR products were analysed by agarose gel electrophoresis. Aliquots of 10µl PCR products were mixed with 2.0 µl of loading dye and loaded on to 1.2% agarose gel and subjected to electrophoresis for 2h at 100 volts in 1 x TAE buffer. Gel was stained with ethidium bromide (0.5µg /ml), destained with distilled water and examined on a UV transilluminator. A 100 bp ladder was used as a molecular size marker. The amplification profile in the gel was documented in a CCD-camera based gel documentation system The template DNA from *Rhizopus* 3spp (2 ) *Aspergillus flavus* (2), *Fusarium* strains (6) and *Aspergillus* (4), *Aspergillus oryzae* (5) strains did not show any amplification. However, toxin producing *Aspergillus flavus* (4) and *Aspergillus parasiticus* (3) showed amplifications.

**The main advantages of the present invention are:**
1. The designed o-methyltransferase, oxidoreductase, and aflatoxin regulatory primers are specific for the detection of aflatoxigenic fungi.
2. The designed primers can detect aflatoxigenic fungi even at 24h of growth.
3. The designed primers can specifically detected fungi possessing aflatoxin-producing genes.
4. A simple and effective use has been used for the isolation of template DNA without the application of liquid nitrogen.

### Sequences of three genes omt, ord A, and afl R.

**A.** O methyltransferase (Omt) gene from the published gene sequence with Accession no **L25834** where the primers covers the region between **1811 to 2218** with the product size **407 bp of SEQ ID NO. 7.**
   **L25834. Aspergillus paras... omt gene [gi:414297]**
**B.** Oxidoreductase (ord) gene from the published gene sequence with Accession no AF **169016** where the primer covers the region between **3142 to 3530** with product size **388 bp of SEQ ID NO. 8.**
   ACCESSION AF169016
   VERSION AF169016.1 GI:6715098 **Aspergillus parasiticus oxidoreductase (ordA), versicolorin B synthase (vbs), cytochrome P450 monooxigenase (cypX), and monooxigenase (moxY) genes, complete cds**
C. Aflotoxin regulatory gene (aflR) from the published gene sequence with Accession no **AF 264763** where the said primer covers the region between 540 to 1338 with the product size 798 bp of SEQ ID NO. 9.
   **Aspergillus sojae strain ATCC 42251 AFLR regulatory protein (aflR) gene, complete cds.**
   ACCESSION AF264763 VERSION AF264763.1 GI:8572226

## Claims

1. One set of oligonucleotide primers of SEQ ID Nos. 1 through 2.

2. A method of identifying aflatoxigenic aspergilli using primers of claim 1 in combination, comprising steps of :
a. Harvesting mixed microflora from food system,
b. Extracting DNA from said harvested flora,
c. Amplifying DNA by PCR using said primers,
d. Analyzing amplified DNA by electrophoresis, and
e. Identifying aflatoxigenic fungi.

3. A method as claimed in claim 2, wherein fungi are harvested by centrifugation.

4. A method as claimed in claim 2, wherein DNA is extracted using mixture of phenol, chloroform, and amyl alcohol in ratio of about 25:24:1.

5. A method as claimed in claim 2, wherein amplification mixture for amplifying DNA comprises Tris-HCl, Potassium Chloride (KCl), Magnesium Chloride (MgCl₂), gelatine, deoxyribonucleoside trisphates, primers, Taq DNA polymerase, and sterile ultra filtered water.

6. A method as claimed in claim 2, wherein electrophorizing amplified DNA uses agarose gel.

7. A method as claimed in claim 2 using three sets of primers of SEQ ID Nos. 1 to 6.

## Patentansprüche

1. Ein Satz von Oligonucleotid-Primern mit den Sequenzidentifizierungsnummern 1 bis 2.

2. Verfahren zum Identifizieren von aflatoxischem Aspergilli,
wobei Primer gemäß Anspruch 1 in Kombination verwendet werden, das die folgenden Schritte aufweist:
a.) Gewinnen einer gemischten Mikroflora aus einem Nahrungsmittelsystem,
b.) Herauslösen der DNA aus der gewonnenen Flora,
c.) Amplifizieren der DNA durch PCR unter Verwendung dieser Primer,
d.) Analysieren der amplifizierten DNA durch Elektrophorese und
e.) Identifizieren von aflatoxischen Pilzen.

3. Verfahren nach Anspruch 2,
wobei die Pilze durch Zentrifugieren gewonnen werden.

4. Verfahren nach Anspruch 2,
wobei die DNA unter Verwendung eines Gemischs von Phenol, Chloroform und Amylalkohol in einem Verhältnis von etwa 25:24:1 herausgelöst wird.

5. Verfahren nach Anspruch 2,
wobei das Amplifizierungsgemisch zum Amplifizieren der DNA Tris-HCl, Kaliumchlorid (KCl), Magnesiumchlorid (MgCl₂), Gelatine, Desoxyribonucleosidtrisphate, Primer, Taq-DNA-Polymerase und steril ultrafiltiertes Wasser aufweist.

6. Verfahren nach Anspruch 2,
wobei die Elektrophoresebehandlung der amplifizierten DNA Agarosegel verwendet.

7. Verfahren nach Anspruch 2,
bei dem drei Sätze von Primern mit den Sequenzidentifierzungsnummern 1 bis 6 verwendet werden.

## Revendications

1. Ensemble d'amorces d'oligonucléotides de SEQ.ID N°1 à 2.

2. Procédé d'identification d'aspergilles aflatoxigènes en utilisant des amorces selon la revendication 1 en combinaison, comprenant les étapes consistant à :
a. récolter la microflore mixte provenant d'un système alimentaire,
b. extraire l'ADN de ladite flore récoltée,
c. amplifier l'ADN par PCR en utilisant lesdites amorces,
d. analyser l'ADN amplifié par électrophorèse, et
e. identifier les champignons aflatoxigènes.

3. Procédé selon la revendication 2, dans lequel les champignons sont récoltés par centrifugation.

4. Procédé selon la revendication 2, dans lequel l'ADN est extrait en utilisant le mélange de phénol, chloroforme et alcool d'amyle en rapport d'environ 25:24:1.

5. Procédé selon la revendication 2, dans lequel le mélange d'amplification pour amplifier l'ADN comprend du Tris-HCl, du chlorure de potassium (KCl), du chlorure de magnésium (MgCl₂), de la gélatine, des trisphates de désoxyribonucléosides, des amorces, une polymérase d'ADN Taq, et de l'eau ultrafiltrée stérile.

6. Procédé selon la revendication 2, dans lequel l'ADN amplifiée par électrophorèse utilise du gel d'agarose.

7. Procédé selon la revendication 2, utilisant trois ensembles d'amorces de SEQ.ID N°1 à 6.


### SEQUENCE LISTING

<110> Council of Scientific and Industrial Research
<120> Novel primers for identifying aflatoxinogenic aspergilli an d an improved use thereof
<130> PCT 185
<140> PCT/IB 02/01162
   <141> 2002-03-26
<160> 6
<170> PatentIn version 3.1
<210> 1
   <211> 20
   <212> DNA
   <213> Artificial Sequence
<220>
   <223> Oligonucleotide forward primer corresponding to gene omt encoding o-methyl transferase.
<400> 1
   agcgtccgaa tccctttaat
   20
<210> 2
   <211> 20
   <212> DNA
   <213> Artificial Sequence
<220>
   <223> Oligonucleotide reverse primer for gene omt encoding o-meth yl tra nsferase
<400> 2
   agggtgttcg ccaatcatag
   20
<210> 3
   <211> 20
   <212> DNA
   <213> Artificial Sequence
<220>
   <223> Oligonucleotide forward primer for gene 'ord' encoding oxidoreduc tase
<400> 3
   actgcccctc agctaacctc
   20
<210> 4
   <211> 20
   <212> DNA
   <213> Artificial Sequence
<220>
   <223> Oligonucleotide reverse primer for gene 'ord' encoding oxidoreduc tase
<400> 4
   gcatcagcat tcttccaagg
   20
<210> 5
   <211> 20
   <212> DNA
   <213> Artificial Sequence
<220>
   <223> Oligonucleotide forward primer for gene 'aflR' encoding aflatoxin regulatory protein
<400> 5
   aaccgcatcc acaatctcat
   20
<210> 6
   <211> 20
   <212> DNA
   <213> Artificial Sequence
<220>
   <223> Oligonucleotide reverse primer for gene "aflR' encoding aflatoxin regulatory protein
<400> 6
   agtgcagttc gctcagaaca
   20